# EUROPÄISCHE PATENTANMELDUNG

(11) **EP 3 725 275 A1**
(43) Veröffentlichungstag der Anmeldung: **21.10.2020**
(21) Anmeldenummer: 19169277.1
(22) Anmeldetag: 15.04.2019
(51) Int. Cl.: A61F 9/00, B01L 3/02, B65D 77/04

(54) **AUFBEWAHRUNGSBEHÄLTER FÜR ZUMINDEST EINE PIPETTE**

(71) Anmelder: Siambanis, Tim, 24576 Weddelbrook (DE)
(72) Erfinder: Siambanis, Tim, 24576 Weddelbrook (DE)
(74) Vertreter: Müller Schupfner & Partner Patent- und Rechtsanwaltspartnerschaft mbB

(57) **Zusammenfassung**

Gegenstand der Erfindung ist ein Aufbewahrungsbehälter (1) mit abnehmbarem Deckel (7) für Pipetten (2) enthaltend eine Behandlungsflüssigkeit, insbesondere eine Behandlungsflüssigkeit für Augen, wobei zumindest eine Pipette im Deckel gehaltert ist sowie ein Set aus dem Aufbewahrungsbehälter und zumindest einer Pipette.

## Beschreibung

Die vorliegende Erfindung betrifft Aufbewahrungsbehälter mit abnehmbarem Deckel für Pipetten enthaltend eine Behandlungsflüssigkeit, insbesondere eine Behandlungsflüssigkeit für Augen, wobei zumindest eine Pipette im Deckel gehaltert ist sowie ein Set aus dem Aufbewahrungsbehälter und zumindest einer Pipette.

### Hintergrund der Erfindung

Zur Behandlung von Augenerkrankungen oder Augenreizungen werden Arzneistoffe oder andere Wirkstoffe lokal im Auge appliziert. Dabei gehören Augensalben und Augentropfen, die als sterile, wässrige bzw. ölige Lösungen oder Suspensionen in den Bindehautsack eingebracht oder auf die Hornhaut getropft werden, zu den gängigsten Behandlungsflüssigkeiten. Die Problematik bei der Anwendung besteht darin, den Wirkstoff auf kleinem Raum sicher, z.B. wegen des Lidschlages, und in der vorgesehenen Menge zu applizieren, um den gewünschten Effekt zu erzielen. Hinzu kommt die notwendige Hygiene zur Vermeidung von Infektionen. Auch trockene Augen sind belastend und benötigen deshalb einer Behandlung mit einer Behandlungsflüssigkeit in Form eines Augenbenetzungsmittels, das den Tränenfilm unterstützt. Insbesondere Personen, die mit krankhaft trockenen Augen an Keratoconjunctivitis Sicca (Trockenes-Auge-Syndrom) leiden, verwenden künstliche Tränenersatzflüssigkeit und dies im Allgemeinen regelmäßig über den Tag verteilt.

Je nach Häufigkeit der Anwendung bieten sich unterschiedliche Behältnisse für den Zweck der Aufbewahrung und Abgabe von Behandlungsflüssigkeiten wie Augentropfen an. Es sind Ein-Dosis-Systeme und Mehr-Dosis-Systeme bekannt. Mehr-Dosis-Systeme sind z.B. Tropffläschchen mit mehreren Millilitern Inhalt. Zu den klassischen Ein-Dosis-Systemen zählen die sogenannten Einzeldosis-Behältnisse. Es handelt es sich hierbei um kleine, durchsichtige oder milchig-trübe Pipetten aus Kunststoff, die die Behandlungsflüssigkeit als sterile Lösung enthalten. Die Verschlusskappe, die die Pipette am vorderen Ende verschließt, wird kurz vor dem ersten Gebrauch abgebrochen bzw. abgedreht.

Am anderen Ende der Pipette befindet sich häufig eine Fahne, mit der die Pipette ergriffen wird. Meistens besitzt die Pipette Wandungen aus weichem Kunststoff, damit der Benutzer mit zwei Fingern einen Druck auf die Abgabekammer der Pipette ausüben kann, der die Abgabe der Behandlungsflüssigkeit aus der Abgabekammer, z.B. in ein Auge, bewirkt.

In der Regel ist nach Anbruch der Pipette die darin enthaltene Behandlungsflüssigkeit nur eine begrenzte Zeit anwendbar, um u.a. eine fortwährende Keimfreiheit zu gewährleisten. Wässrige Zubereitungen in Mehr-Dosis-Systeme sind meist mit Zusatzstoffen konserviert und dürfen nach Anbruch noch einige Zeit, z.B. 1 bis 4 Wochen, teilweise auch wesentlich länger, entsprechend den Herstellerinformationen benutzt werden.

Eine Zugabe von Konservierungsmitteln ist bei Ein-Dosis-Systemen i.d.R. - z.B. wegen möglicher Nebenwirkungen - nicht erwünscht bzw. nicht erforderlich, da keine Keime in die geschlossene Pipette eindringen können. Die Pipette wird in der Regel nach einmaliger Anwendung weggeworfen, womit diese Anwendung allerdings nicht ressourcensparend ist. Es ist jedoch oft nicht zwingend notwendig, die Pipette unmittelbar nach dem ersten Gebrauch zu entsorgen, wenn nach der ersten Anwendung noch Behandlungsflüssigkeit für weitere Anwendungen verbleibt und andererseits die Gefahr einer veränderten Wirkung oder einer Verkeimung innerhalb des relativ kurzen zeitlichen Abstands bis zu den darauf folgenden Anwendungen gering ist bzw. ausgeschlossen erscheint, zumindest wenn diese bis dahin sachgerecht aufbewahrt werden.

Laut vieler Fachstimmen kann eine Pipette mit Tränenersatzflüssigkeit nach dem Öffnen durch Abdrehen der Verschlusskappe noch etwa 24 Stunden verwendet werden, bevor die Keimbelastung durch die überwiegend wässerigen Lösungen bedenklich werden würde. Somit kann mehrmals am Tag Tränenersatzflüssigkeit aus ein und derselben Pipette ins Auge getropft werden.

Von Ärzten und Apothekern wird gelegentlich empfohlen, die Pipette nach dem ersten Gebrauch zum Schutz in Aluminiumfolie einzuwickeln. Dies ist jedoch unpraktisch in der Handhabung und der Kontakt der offenen Pipettenspitze bzw. der Tropfenflüssigkeit mit dem Aluminium wird in Kauf genommen. Außerdem fließt oft Flüssigkeit aus den Pipetten, wenn Druck - z.B. in der Hosentasche - auf die Pipette ausgeübt wird.

Es sind auch schon Aufbewahrungsbehälter für Pipetten enthaltend Augentropfen vorgeschlagen worden. Das Deutsche Gebrauchsmuster DE 29508042 U1 offenbart Aufbewahrungsbehälter mit Deckel zum Aufstecken oder Aufschrauben auf die Gehäuseöffnung. Der Aufbewahrungsbehälter soll eine Pipette aufrechtstehend aufnehmen, wobei die Abgabeöffnung der Pipette zum Deckel gerichtet ist. Ähnlicher Natur ist der Gegenstand der US 2008/0283530 A1. Dort wird aber ein zweiteiliger nach oben offener Aufbewahrungsbehälter offenbart, in den die Pipette verschlossen mit der Abgabeöffnung nach oben gestellt wird.

### Aufgabe der vorliegenden Erfindung

Die vorliegende Erfindung hat es sich daher zur Aufgabe gemacht, eine Pipette, enthaltend eine Behandlungsflüssigkeit, sowohl im ungeöffneten als auch im geöffneten oder im wiederverschlossenen Zustand sicher vor biologischen, chemischen oder physikalischen Beeinträchtigungen geschützt aufbewahren zu können. Desweiteren soll die Aufbewahrungsmöglichkeit eine leichte Handhabung im Allgemeinen und speziell der Pipette ermöglichen, insbesondere bei der Anwendung der Behandlungsflüssigkeit als Augentropfen, und die Anwendbarkeitsdauer der Behandlungsflüssigkeit in der jeweiligen Pipette über die Einmalanwendung hinaus verlängern.

### Zusammenfassung der vorliegenden Erfindung

Die vorliegende Erfindung betrifft einen Aufbewahrungsbehälter für zumindest eine geöffnete oder geschlossene Pipette mit Fahne, wobei die Fahne ein Ende der Pipette bildet und die Fahne ein Fahnenende aufweist, wobei der Aufbewahrungsbehälter aufweist:
- ein rohrförmiges Gehäuse, das an seinem ersten Ende eine erste Öffnung aufweist und
- einen ersten Deckel zum Verschließen und Öffnen der ersten Öffnung des Gehäuses, wobei der Deckel auf seiner der Öffnung des Gehäuses zugewandten Seite eine Aufnahme für das Fahnenende der Pipette zum Haltern der zumindest einen Pipette am Deckel aufweist,
und ein Set aus Aufbewahrungsbehälter und Pipette gemäß Anspruch 10.

Das rohrförmige Gehäuse hat insbesondere die Form eines länglichen, geraden Rohres.

Bevorzugte Ausführungsformen sind Gegenstand der Unteransprüche oder nachfolgend beschrieben.

Nach einer Ausführungsform ist das dem ersten Ende gegenüberliegende, zweite Ende des rohrförmigen Gehäuses durch einen Gehäuseboden als integraler Bestandteil des Gehäuses dauerhaft verschlossen.

In einer weiteren Ausführungsform weist das rohrförmige Gehäuse an seinem zweiten Ende eine zweite Öffnung auf, wobei ein zweiter Deckel zum Verschließen und Öffnen der zweiten Öffnung oder andererseits ein an der zweiten Öffnung befestigter, dauerhaft verschließender Gehäuseboden vorgesehen ist. Der zweite Deckel weist nach einer Ausführungsform auf seiner der zweiten Öffnung des Gehäuses zugewandten Seite eine Aufnahme für das Fahnenende einer Pipette zum Haltern der Pipette auf, wobei die Pipette die zweite Pipette im Gehäuse sein kann oder die erste Pipette, wobei es sich jeweils auch um eine alternative Art der Aufnahme (zur ersten Aufnahme) für ein anders ausgebildetes Fahnenende handeln kann.

Ist eine Pipette im einem Deckel gehaltert und verschließt der Deckel das Ende des Gehäuses, erstreckt sich die Längsachse der Pipette im Wesentlichen parallel zur Längsachse des rohrförmigen Gehäuses, wobei das vordere Ende der Pipette je nach Ausführungsform des Behälters - weder Gehäuseboden oder Deckel des anderen Gehäuseendes noch das vordere Ende einer etwaigen zweiten, von dem anderen Ende her in das Gehäuse eingesetzten Pipette erreicht.

Nach einer bevorzugten Ausführungsform erstreckt sich die Pipette, wenn im ersten oder zweiten Deckel gehaltert, abgesehen von etwaigen seitlichen Führungen im Inneren des Gehäuses, vorne, d.h. zumindest ab dem vorderen Ende der Aufnahme, im Wesentlichen freischwebend.

In einer weiteren Ausführungsform werden zwei Pipetten im Wesentlichen parallel nebeneinander über deren jeweiliges Fahnenende in einer oder zwei Aufnahmen des ersten Deckels gehaltert, wobei das zweite Ende des Gehäuses einen Gehäuseboden aufweist. Die beiden Pipetten können in Form eines Streifens aus zwei Pipetten miteinander verbunden sein oder einzeln in die Aufnahme geschoben sein.

### Detaillierte Beschreibung der Erfindung

Die in dem Aufbewahrungsbehälter nach der vorliegenden Erfindung einsetzbare Pipette umfasst zumindest
- eine Fahne als leicht greifbares, hinteres Ende der Pipette, wobei die Fahne ein Fahnenende aufweist,
- eine Abgabekammer, in der sich die Behandlungsflüssigkeit, z.B. die Augentropfen, befinden,
- eine Abgabeöffnung zum Ausgeben der Behandlungsflüssigkeit aus der Abgabekammer, und
- eine Verschlusskappe für die Abgabeöffnung am vorderen Ende der Pipette.

Das hintere Ende der Pipette wird von der Fahne und das gegenüberliegende, vordere Ende der Pipette von der Verschlusskappe gebildet. Die dazwischen liegende Abgabekammer endet vorzugsweise an ihrem vorderen Ende mit einem Halsstück, welches die Abgabeöffnung aufweist, die durch die Verschlusskappe verschlossen ist, und nach außen, zum vorderen Ende hin, die Pipettenspitze bildet. Die Abgabekammer geht an ihrem hinteren Ende in die Fahne über. Das Fahnenende sei definiert als der hintere Bereich der Fahne, der von der Aufnahme zur Halterung der Pipette benötigt wird, wobei sich das Fahnenende auch bis zum vorderen Ende der Fahne erstrecken kann.

Die Pipetten sind vorzugsweise aus einem Kunststoffmaterial, insbesondere aus PE oder aus PP, gefertigt.

Meistens sind die Pipetten länglich und flach ausgebildet. Sie weisen i.d.R. zumindest im Bereich der Fahne zwei parallele Längsseiten und eine in der Regel bauchige Abgabekammer auf, die sich beidseits der durch die großen Fahnenflächen gebildeten Ebenen erhebt. In der Abgabekammer der Pipette befindet sich i.d.R. ein Inhalt von ca. 0,1 bis 1,5 ml, häufig 0,2 bis 0,6 ml.

Die Fahne hat eine Plättchenform. Das Plättchen hat z.B. die Form eines dünnen länglichen Quaders. Die Fahne kann eine oder mehrere Produkt-Kennzeichnungen aufnehmen. Die Produkt-Kennzeichnung ist i.d.R. auf die Fahne in Form einer Etikette aufgeklebt. Es kann aber auch eine Kennzeichnung in die Fahne eingeprägt oder aufgedruckt sein. Gleichzeitig dient die Fahne als Anfasshilfe.

Vielen gebräuchlichen, auf dem Markt befindlichen Pipetten ist gemeinsam, dass sie zur besseren Handhabung ein solches flaches, quaderförmiges Endstück besitzen, um die Pipette einfach greifen zu können. Die Fahne, insbesondere das Fahnenende, weist über viele Hersteller hinweg relativ einheitliche Abmessungen hinsichtlich der Breite (typischerweise 1,2 - 1,3 cm), der Stärke (typischerweise 0,2 bis 0,4 cm) und der Länge (typischerweise 2 bis 3 cm) der Fahne auf, mit nur geringen, herstellerabhängigen Abweichungen. Deshalb ist eine standardisierte Aufnahme für das Fahnenende für eine große Anzahl an unterschiedlichen Pipetten realisierbar.

In der Regel ist die Fahne hohl. Die beiden Folienwände seiner großen Quaderseiten weisen i.d.R. einen konstanten Abstand auf und sind nur im Randbereich dieser Quaderseiten miteinander verschweißt. Weniger häufig sind Pipetten anzutreffen, bei denen beide Folienwände im Bereich der Fahne miteinander vollflächig verschweißt oder verklebt sind.

Pipetten mit Behandlungsflüssigkeit werden i.d.R. in Form von einer Mehrzahl von Pipetten, verknüpft jeweils untereinander zu einem sogenannten Streifen, vertrieben. Der Streifen hat die Form einer längsseitigen Aneinanderreihung mehrerer Pipetten. Die Pipetten weisen hierfür Randbereiche (z.B. auch in Form von Verbindungsstegen) auf, welche miteinander entlang von Sollbruchlinien verbunden sind. Die Sollbruchlinien verlaufen insbesondere beidseits entlang der schmalen Längsseite der Fahnen, insbesondere parallel.

Die Anwendung einer Pipette, am Bespiel der Anwendung für ein Auge erläutert, erfolgt in der Regel wie folgt. Die Pipette wird von dem Streifen abgebrochen. Hierzu fasst man die Pipette am besten im Bereich der Fahne an und bricht eine endständige Pipette vom Streifen ab.

Wenn gewünscht, kann man die Pipette einige Male mit der Hand nach unten schlagen, bis sich die Flüssigkeit auch im Halsstück der Pipette nahe der Abgabeöffnung befindet. Nun dreht man die Verschlusskappe der Pipette ab und hält die Pipette zum Eintropfen zwischen Zeigefinger und Daumen über das Auge. Durch Druckausüben auf die Abgabekammer entweicht die Behandlungsflüssigkeit über die Abgabeöffnung, in das Auge.

Den Pipetten ist gemeinsam, dass diese ohne Druckausüben auf die Abgabekammer nicht auslaufen, auch nicht, wenn diese bereits teilentleert sind und die Abgabeöffnung freischwebend nach unten hängt.

Bei vielen Pipetten kann die Verschlusskappe nach dem erstmaligen Entfernen auf die Abgabeöffnung wieder aufgesetzt werden, und diese ist so wieder verschließbar. Es sind jedoch auch Pipetten erhältlich, wo die Verschlusskappe nicht wieder auf die Abgabeöffnung aufgesetzt werden kann.

Nach einer Ausgestaltung kann die Verschlusskappe ein Griffstück und den eigentlichen Verschluss aufweisen, wobei das Halsstück der Abgabekammer über verformbare Stege zusätzlich mit dem Griffstück verbunden sein kann. Der Verschluss steht auf der in Richtung der Abgabeöffnung weisenden Seite des Griffstücks hervor und ist oft mit einer halbkugelförmigen, hohlen Aufweitung versehen, welche geeignet ist, die ringförmige Pipettenspitze durch ein engeres, flexibles Eingangsloch der Halbkugel-Grundfläche zu stoßen und über die Presspassung der Lochwandung mit dem Halsstück zu verschließen. Andere Ausgestaltungen haben zusätzlich im höchsten Punkt der Halbkugelkuppel einen Stift, der mit einer engen Passung in die Abgabeöffnung eingeführt wird.

Nebeneinander liegende Griffstücke können Ihrerseits auf Sollbruchlinien durch Stege miteinander verbunden sein. Die Sollbruchlinien der Abgabekammern bzw. die Sollbruchlinien entlang der schmalen Längsseite der Fahnen liegen vorzugsweise auf einer Geraden mit den Sollbruchlinien der Griffstücke. Vielfach sind die Griffstücke jedoch nicht mit benachbarten Griffstücken verbunden, sondern sind jeweils separat ausgebildet.

Die Griffstücke erlauben es, die Verschlusskappen leichter abzubrechen bzw. abzudrehen, ohne diese zu verlieren und die Verschlusskappen nach der Anwendung wieder auf die Pipettenspitzen aufzustecken. Meistens ist jedoch eine Handhabung durch die kleine Dimensionierung der Verschlusskappen für den Anwender schwierig, zumindest sehr unpraktisch und anfällig für Verunreinigungen.

Der hier beschriebene Aufbewahrungsbehälter dient zur Aufbewahrung von einer Pipette oder in weiteren Ausführungsformen von einer oder zweier Pipetten mit Fahne, wobei die Pipetten ungeöffnet, geöffnet oder wiederverschlossen in dem Behälter aufbewahrt werden können.

Der Behälter umfasst ein Gehäuse in Form eines länglichen, geraden Rohrs. Das Gehäuse besitzt am ersten Ende eine erste Öffnung, die mit einem ersten Deckel verschlossen oder geöffnet wird.

Das Gehäuse weist an seinem zweiten Ende einen Gehäuseboden als integraler Bestandteil des Gehäuses auf oder besitzt dort eine zweite Öffnung, die mit einem Gehäuseboden als separates Bauteil dauerhaft verschlossen ist oder die mit einem zweiten Deckel verschlossen oder geöffnet wird.

Ein Deckel als Teil des erfindungsgemäßen Behälters ist dazu bestimmt, regelmäßig zum Öffnen bzw. Verschließen des Gehäuses abgenommen bzw. aufgesetzt zu werden. Ein Gehäuseboden ist dazu bestimmt, nach dem Zusammenbau mit dem Gehäuse nicht mehr von dem Gehäuse abgenommen zu werden.

Der erste Deckel weist, auf seiner der Öffnung des Gehäuses zugewandten Seite, eine Aufnahme für das Fahnenende zum Haltern einer Pipette am Deckel auf oder weist in weiteren Ausgestaltungen eine breite Aufnahme für bis zu zwei Pipetten oder zwei Aufnahmen für jeweils eine Pipette auf.

Der zweite Deckel kann, je nach Ausführungsform des Behälters, eine Aufnahme für eine Pipette aufweisen oder sieht keine Aufnahme vor und dient dann am zweiten Ende des Gehäuses z.B. nur dem Verschluss oder zum Öffnen und Verschließen bei etwaigen Reinigungen des Gehäuses.

Ein Deckel mit Aufnahme kann als ein Teil oder aus zusammengefügten Teilen gefertigt sein. Bei Letzterem können die ganze Aufnahme oder Aufnahmekomponenten vom Deckel wieder lösbar (z.B. durch einen Schnapp- oder Schraubverbindung) oder fest verbunden (z.B. durch Schweißen, Löten, Kleben, Einpressen, Verformen) sein.

Im Gebrauchszustand des Aufbewahrungsbehälters können über seine erste Öffnung oder ggf. über die erste und zweite Öffnung bis zu zwei Pipetten mit Hilfe von einem Deckel mit einfacher oder doppelter Aufnahme (für eine Pipette oder bis zu zwei Pipetten) oder mit Hilfe von zwei Deckeln jeweils mit Aufnahme (für jeweils eine mögliche Pipette) in das Gehäuse eingeführt bzw. aus dem Gehäuse herausgezogen werden. Die Aufnahme der jeweiligen Pipette erfolgt auf der am Behälter innenliegenden Seite des Deckels und hält die Pipette über deren Fahnenende am Deckel fest. Die Deckel erlauben neben der Pipettenhalterung auch den Verschluss oder das Öffnen des Gehäuses im häufigen, täglichen Gebrauch. Die Deckel sind also mit oder ohne Pipette von dem Gehäuse abnehmbar und ohne das Gehäuse beweglich.

Das Einführen der Pipette in das Gehäuse, die in der Aufnahme eines Deckel gehaltert ist, erfolgt vorzugsweise durch Anschlagen bzw. Ansetzen des Pipettenhalsstücks an den Rand der Gehäuseöffnung mit anschließender Rotation der Pipette um die Kontaktlinie, so dass sich die Pipettenspitze in das Gehäuse hineindreht und die Längsachsen von Pipette und Gehäuse im Wesentlichen parallel ausgerichtet werden. Danach kann die Pipette ganz in das Gehäuse eingeschoben werden. Generell kann die Einführung der Pipette bei einer gewöhnlich ruhigen Hand auch direkt, d.h. ohne Anschlag bzw. Ansetzen erfolgen, wobei die Längsachsen der Pipette und des Gehäuses dann von vornherein im Wesentlichen übereinstimmen.

Das Gehäuse hat einen vorzugsweise kreisrunden, konstanten Querschnitt, kann aber auch einen beliebigen Querschnitt und Querschnittsverlauf in Längsrichtung haben, wie beispielweise einen vieleckigen Querschnitt, etwa in Form eines Achtecks oder einen Querschnitt mit zwei längeren Parallelen, die durch Halbkreise miteinander verbunden sind, so dass zwei Pipetten nebeneinander in das Gehäuse geschoben werden können.

Der Aufbewahrungsbehälter hat typischerweise, unabhängig von den unterschiedlichen oben beschriebenen Bauformen, eine Länge im Bereich von 3 bis 15 cm und eine Breite im Bereich von 1 bis 4 cm. Der Aufbewahrungsbehälter ist hinsichtlich Länge und Breite für die unterschiedlichen Bauformen vorzugsweise wie folgt durch eines oder mehrere der folgenden Merkmale gekennzeichnet:
a) Die Länge des Aufbewahrungsbehälters beträgt 3 bis 15 cm, insbesondere 5 bis 9 cm, wenn der Aufbewahrungsbehälter für eine oder zwei im selben Deckel gehaltene Pipetten vorgesehen ist, oder 7 bis 15 cm, insbesondere 8 bis 11 cm, wenn der Aufbewahrungsbehälter für zwei im Wesentlichen entlang der Längsachse des rohrförmigen Gehäuses angeordnete Pipetten vorgesehen ist.
b) Wenn Aufbewahrungsbehälter im ersten Deckeln nur eine Aufnahme für eine Pipetten aufweist, beträgt die Breite des Aufbewahrungsbehälters 1 bis 3 cm, insbesondere 1,3 bis 2 cm, wenn der Aufbewahrungsbehälter einwandig ist, bzw. 2 bis 2,6 cm, wenn der Aufbewahrungsbehälter doppelwandig ist.
c) Wenn der Aufbewahrungsbehälter im ersten Deckeln eine Aufnahme für zwei Pipetten oder zwei Aufnahmen für jeweils eine Pipette aufweist beträgt die Breite des Aufbewahrungsbehälters 2 bis 4 cm, insbesondere 2,5 bis 3,3 cm, wenn der Aufbewahrungsbehälter einwandig ist, bzw. 3,3 bis 3,9 cm wenn der Aufbewahrungsbehälter doppelwandig ist.

Bei der Ausführungsform, in der bis zu zwei Pipetten im ersten Deckel gehaltert sind, erhöht sich die Breite des Behälters gegenüber der Bauform mit nur einer Pipette im ersten Deckel in zumindest einer Querrichtung um z.B. ca.1,3 cm. Die Behälterlänge liegt eher im unteren der oben angegeben Bereiche, wenn der Anwender nur Pipetten ohne Verschlusskappe im Behälter aufbewahren möchte und/oder wenn er die Fahne an Ihrem hinteren Ende auf eine zur Halterung minimal benötigte, verbleibende Länge, insbesondere 0,5 bis 1 cm, kürzt.

Nach einer Ausführungsform nimmt der Behälter eine einzige Pipette über die Öffnung an einem Gehäuseende auf, während das zweite Gehäuseende durch den Gehäuseboden als z.B. integralen Bestandteil des Gehäuses oder einen zweiten Deckel (ggf. ohne Aufnahme) verschlossen ist. Ein zweiter Deckel ohne Aufnahme erleichtert die Reinigung des Gehäuses, hat aber darüber hinaus keine besondere Funktion.

Statt eines Aufbewahrungsbehälters für nur eine einzige Pipette ist ein Aufbewahrungsbehälter für bis zu zwei Pipetten gelegentlich praktischer, wenn z.B. der Inhalt einer Pipette als Tagesdosis nicht ausreicht oder zwei unterschiedliche fließfähige Behandlungsmedien einzusetzen sind. Sehr trockene Augen, öfter einfallende Wimpern durch Lidrand-Entzündungen, starker Wind, usw. verlangen häufig eine höhere Tropffrequenz, womit der Inhalt einer einzigen Pipette am Tag nicht ausreicht. Dafür kann der Behälter nach einer Ausführungsform zwei Pipetten über die beiden gegenüberliegenden Öffnungen des rohrförmigen Gehäuses mit Hilfe von Gehäusedeckeln mit integrierter Aufnahme aufnehmen. Jeder der beiden Deckel haltert dann eine Pipette bzw. ist zur Halterung jeweils einer Pipette ausgebildet und verschließt das Gehäuse an dem jeweiligen Ende in einer wieder zu öffnenden Form. Die Konstruktion der beiden Deckel kann identisch oder abweichend voneinander sein. So kann der zweite Deckel im Wesentlichen die gleiche Form (wie z.B. eine Topfform mit Innengewinde) wie der erste Deckel haben, aber z.B. keine Aufnahme zum Haltern einer zweiten Pipette. Die Aufnahme im zweiten Deckel kann gleich der Aufnahme im ersten Deckel sein oder unterschiedlich ausgebildet zur Aufnahme und Halterung anders geformter Fahnenenden sein. So kann z.B. neben einer Pipette mit Tränenersatzflüssigkeit auch eine Pipette mit einer Kochsalzlösung zum Spülen der Augen in dem Aufbewahrungsbehälter aufbewahrt werden.

Zwei Pipetten können in einer alternativen Ausführungsform auch direkt an dem ersten Deckel nebeneinander über eine breitere, gemeinsame Aufnahme oder zwei getrennte Aufnahmen jeweils für zwei mögliche Fahnenenden gehaltert werden, wobei der Gehäusequerschnitt entsprechend größer ausgelegt ist und das zweite Gehäuseende einen Gehäuseboden oder einen zweiten Deckel ohne Aufnahme aufweist. Die beiden Pipetten sind an ihren schmalen Längsseiten getrennt oder mit ihren ursprünglichen Verbindungsstegen verbunden. Diese Variante erlaubt wie die vorherige Ausführungsform auch die Halterung von nur einer einzigen Pipette im Aufbewahrungsbehälter.

Ist bei allen Ausführungsformen eine Pipette im Deckel gehaltert und der jeweilige Deckel verschließt die zugehörige Öffnung des Gehäuses, erstreckt sich die Längsachse der Pipette im Wesentlichen parallel zur Längsachse des rohrförmigen Gehäuses, wobei das vordere Ende der Pipette - je nach Behältervariante - weder Gehäuseboden oder Deckel des anderen Gehäuseendes noch das vordere Ende einer etwaigen zweiten, von dem anderen Ende her in das Gehäuse eingesetzten Pipette erreicht.

Nach einer bevorzugten Ausführungsform erstreckt sich die Pipette, wenn im Deckel gehaltert, abgesehen von etwaigen seitlichen Führungen im Inneren des Gehäuses, vorne, d.h. zumindest ab dem vorderen Ende der Aufnahme, im Wesentlichen freischwebend.

Der Deckel kann mit seiner vorderen Seite, d.h. auf seiner der Öffnung des Gehäuses zugewandten Seite, auf seine zugehörige Öffnung z.B. aufgeschoben, aufgesteckt, aufgedrückt, eingerastet oder aufgeschraubt werden. Insbesondere weist der Deckel ein Innen- oder ein Außengewinde auf, das jeweils mit einem zugehörigen Gewinde am Gehäuseende korrespondiert.

Die nach außen gewandte Seite des Deckels mit Aufnahme kann so konstruiert sein, dass der Deckel auf seine zugehörige Gehäuseöffnung oder am gegenüberliegenden Gehäuseende auf den Gehäuseboden oder auf den zweiten Deckel, z.B. in Form einer Steckpaarung, ähnlich wie bei der Kappe eines Füllfederhalters, aufgesetzt werden kann.

Nach einer Ausgestaltung ist der Deckel topfförmig ausgebildet und weist in seinem Topfinneren die Aufnahme für das Fahnenende der Pipette auf, die z.B. über einen Klemmmechanismus das Fahnenende der Pipette aufnimmt und festhält.

Der Deckel kann vorzugsweise mit der Pipette, das Fahnenende in der Aufnahme, auf eine Ablagefläche, wie z.B. auf einen Tisch, auf seiner am Behälter außenstehenden Seite sicher abgelegt werden, ohne dass die Pipettenspitze die Ablagefläche berührt, insbesondere z.B. liegt ein topfförmiger Deckel auf seiner äußeren Deckelbodenfläche auf, wobei die Längsachse der Pipette senkrecht zur Ablagefläche und die Pipettenspitze nach oben ausgerichtet sind.

Das Risiko des Kontakts der Pipettenspitze mit der Ablagefläche ist somit geringer als bei der Handhabung der Pipette ohne Deckel. Die Standfestigkeit kann durch einen niedrigen Schwerpunkt des Deckels nahe dem Deckelboden verbessert werden. Dies kann mit einem verstärkten Deckelboden mit deckeleigenem Material oder mit einem zusätzlichen, an dem Deckelboden befestigten Element, z.B. in Form einer im Deckel liegenden Metallringscheibe bei einer Topfform des Deckels, erreicht werden. Zudem kann ein Dauermagnet, insbesondere ein Magnet umfassend Seltenerdmetall, wie z.B. Sm₂Co₁₇, am Deckelboden angebracht werden, der den Schwerpunkt weiter zum Deckelboden hin verschiebt und das Anhaften des Deckels an ferromagnetischen Gegenständen des Alltags erlaubt.

Beim Tropfen ist es möglich, den Deckel an der verwendeten, aufgenommenen Pipette zu belassen.

Zahlreiche Methoden zum Aufnehmen, Ausrichten und Festhalten des Fahnenendes der Pipette in der Aufnahme am Deckel sind möglich: Kniehebel oder Schraubklemmen, die die große Fahnenfläche gegen einen Führungsanschlag drücken oder eine spitze Schraube, die sich in die Fahne hineindrückt und gleichzeitig die Fahne gegen einen Führungsanschlag presst.

Einfache Lösungen sind die Verwendung von Federkonstruktionen oder die Ausnutzung der elastischen und/oder plastischen Eigenschaften der Fahne selbst. Das Fahnenende wird dabei in die Aufnahme am Deckel hineingeschoben und festgeklemmt.

Die Aufnahme kann in Form einer Federkonstruktion ausgebildet sein. Bei der Federkonstruktion kann zumindest eine Federwange die Fahne in Richtungen ihrer Breite B und zumindest eine Federwange in Richtung der Stärke S ausrichten, so dass die Längsachse der Pipette in Längsrichtung des Deckels bzw. in Längsrichtung des Gehäuses ausgerichtet wird. Aufgrund der geringen herstellerabhängigen Abweichungen der Pipettenbreite B kann i.d.R. auf die Federwange(n) in Breitenrichtung auch verzichtet werden und mit zwei einfachen Führungsflächen für die schmalen Längsseiten gearbeitet werden. Diese Führungsflächen können an der Federkonstruktion, aber auch an der am Behälter innenliegenden Deckelwand realisiert sein, z.B. in Form einer Extrusion oder einer fließgepressten Protrusion des Bodens eines topfförmigen Deckels. In Richtung der Fahnenstärke S kann mit einer Paarung aus zwei geschwungenen, gleichförmigen Federwangen oder einer Paarung aus einer geschwungenen Federwange mit einem ebenen Führungsanschlag gearbeitet werden, ähnlich einem Papierclip. Die Biegeebene der Federwange kann beliebig - insbesondere aber vertikal oder parallel - zur Deckellängsachse liegen. Der Kontakt der Federwange mit der Fahne ist in der Regel linienförmig, kann aber auch punkt- oder flächenförmig sein. Die Federwange liegt i.d.R. auf der Fahne elastisch auf, kann aber auch die Fahne plastisch verformen, z.B. durch eine nadelförmige Spitze an der Kontaktlinie der Federwange zur Fahne.

Das Material der Aufnahme sollte idealerweise aus dem gleichen oder ähnlichem Material wie das Deckelmaterial sein, um Fügeverfahren wie das Schweißen oder Löten leichter zu ermöglichen und eine galvanische Korrosion zwischen Aufnahme oder Teilen der Aufnahme und Deckel bei direktem Kontakt zu vermeiden, ohne dagegen Vorsichtsmaßnahmen treffen zu müssen, z.B. durch Anbringung von Trennschichten oder durch eine Abdichtung.

Die Verwendung anderer Materialen für die Federwange als das Material des Deckels können in Betracht gezogen, dies sind insbesondere hochfeste Federmaterialien wie z.B. Titanlegierungen oder Edelstähle.

Zur Umgehung der Konstruktion einer separaten im Deckel anzubringenden Federkonstruktion zur Aufnahme des Fahnenendes kann z.B. die Federkonstruktion als integraler Bestandteil des Deckels im Spritzguss- oder Fließpressverfahren gefertigt werden.

Außerdem können zur Vermeidung der Verwendung von Federwangen in der Konstruktion der Aufnahme die Eigenschaften der Fahne in Form von elastischer und/oder plastischer Verformbarkeit genutzt werden.

Eine elastische Verformung entsteht zum einen an der Fahne beim Zusammendrücken der großen Fahnenflächen parallel zur Richtung der Stärke S aufgrund des hohlen Fahnenkörpers.

Dies kann nach einer ersten Bauform ausgenutzt werden, indem z.B. die Fahne zum Haltern in eine quaderförmige Extrusion als Maul am Deckelboden eines topfförmigen Deckels eingeschoben wird, deren Öffnung dem Fahnenquerschnitt angepasst ist und die in Richtung der Stärke S eine klemmende Verengung aufweist. Die Verengung ist dabei kleiner als die Stärke S der Fahne, um eine Federwirkung an den großen Fahnenflächen zu erzielen.

Nach einer zweiten Bauform kann durch elastisches Biegen der Fahne um eine Achse parallel zur Richtung der Fahnenbreite B eine Haltekraft erzeugt werden. Der Deckel kann dabei, wie zuvor beschrieben, eine quaderförmige Extrusion am Deckelboden haben.

Die Extrusionsrichtung besitzt hier jedoch eine bogenförmige Krümmung, wodurch beim Einschieben der Fahne (durch die Extrusionsöffnung) die Wand der Extrusion eine Biegekraft auf die Fahne ausübt. Dadurch entsteht wiederum ein Reibschluss an den großen Fahnenflächen, die die Pipette in dieser Aufnahme hält.

Neben den elastischen Eigenschaften, wie bei den beiden vorherigen Bauformen, lassen sich, wie z.B. bei den folgenden zwei Bauformen, ggf. auch die plastischen Eigenschaften der Fahne ausnutzen.

Nach einer dritten Bauform können z.B. keilförmige Verengungen, ähnlich der ersten Bauform, vorgesehen sein, die sich jedoch ganz am Ende der Extrusion befinden und sich dort als Verjüngung der Aufnahme in die hinteren Kanten der Fahne hinein treiben.

Nach einer vierten Bauform kann die Aufnahme, ähnlich wie bei der zweiten Bauform, eine oder hier aber auch mehrere Krümmungen der Fahne (um Achsen parallel zur Richtung der Breite B entlang der Aufnahmerichtung) erzwingen. Weiterhin können hier die Krümmungen so gestaltet sein, dass entweder nur eine elastische Verformung oder eine elastische und plastische Verformung der Fahne durch Überschreiten der Streck- oder Fließgrenze (mit entsprechend vergleichsweise kleineren Biegeradien bzw. größeren Neigungswinkeln und demzufolge höheren Haltekräften) eintritt. Wird z.B. in einem topfförmigen Deckel ein gerader Quader aus dem Deckelboden extrudiert und sind die beiden großen Quaderseiten - abwechselnd in Aufnahmerichtung - mit Vertiefungen in den Quaderinnenraum hinein (also in Richtung der Fahnenstärke S) versehen, so dass sich ein wellenförmiger oder S-förmiger Eingang für die Fahne ergibt, dann findet eine Klemmung der Fahne durch Reibschluss aufgrund der Biegekräfte, die die Vertiefungen erzeugen, statt.

Zum Haltern des Fahnenendes mit den vier oben genannten Bauformen sind - statt der geschilderten Extrusion des Deckelbodens in der am Behälter nach außen weisenden Richtung - auch Aufnahmen möglich, die sich wie bei der Federkonstruktion in den Gehäuseinnenraum hinein erstrecken. Auch kann eine Aufnahme, die das Fahnenende nach diesen Bauformen haltert, als vom Grundkörper des Deckels getrenntes Teil gefertigt werden, das dann später am Deckel befestigt wird.

Die Aufnahmen, wie die drei oben genannten Bauformen zum Haltern des Fahnenendes, besitzen keine beweglichen Komponenten wie z.B. Federwangen oder Schrauben. Aufnahmen, die keine beweglichen Komponenten zum Haltern des Fahnenendes besitzen, werden in der vorliegenden Erfindung auch als Maul bezeichnet.

Der Ausbewahrungsbehälter sollte möglichst leicht und klein sein, damit die Handhabung wie z.B. das Tragen oder Anwenden der Pipette für den Benutzer angenehm bleibt. In dieser Hinsicht können Versteifungen das Risiko eines Beulens bei dünnen Behälterwänden minimieren. Reichen diese Versteifungen, z.B. bei einem kreisrunden Querschnitt des Gehäuses in Form von radial eingedrückten, kreisförmigen Rillen, in den Innenraum des Gehäuses, dann können sie, bei angepasstem Innendurchmesser, gleichzeitig zur besseren Führung beim Herausziehen bzw. Hineinschieben der Pipette dienen. Solche Rillen können desweiteren dazu dienen, die Gesamtkonstruktion des Behälters durch eine Tragevorrichtung - z.B. in Form eines zusätzlichen Clips - an Kleidungsteilen oder Taschen zu fixieren.

Zum Schutz gegen eine unerwünschte Änderung der Temperatur der Behandlungsflüssigkeit kann der Aufbewahrungsbehälter in einer Ausführungsform thermisch isoliert werden. Dies kann bei sommerlichen Temperaturen oder beim Tragen des Behälters nah am Körper vorteilhaft sein. So entsteht z.B. beim Tragen eines nicht isolierten Behälters in der Hosentasche der Nachteil, dass die Augentropfen die Körpertemperatur annehmen und deswegen beim Eintropfen kaum gespürt werden können. Zu kühle Augentropfen werden wiederum als sehr unangenehm empfunden. Außerdem sind z.B. Pufferlösungen von Augentropfen in Ihrer Qualität temperaturabhängig. Von den meisten Herstellern von Augentropfen wird eine maximale Lagertemperatur von 25°C angegeben, die es möglichst auch bei der Behälterkonstruktion zu berücksichtigen gilt, auch wenn nach Stimmen aus der Fachwelt diese Temperatur kurzfristig überschritten werden darf.

Zur thermischen Isolierung kann ein doppel- oder mehrwandiger Aufbewahrungsbehälter dienen, dessen Isolierungsstärke insbesondere durch die Wahl eines Behältermaterials mit geringer Wärmeleitung (z.B. Edelstahl oder Titanlegierung statt Aluminium), eines effektiven Dämmmaterials (z.B. Schaum, Pulver, Fasern, Pulver mit Metallzusatz, Vielschichtisolierung) und durch ein Vakuum beeinflusst werden kann.

In einer Ausführungsform wird der Aufbewahrungsbehälter doppelwandig mit eingeschlossenem Vakuum konstruiert. Bei der Isolierung eines solchen doppelwandigen Behälters durch ein Vakuum (im Raum zwischen Außen- und Innenwand) liegt der Druck im Bereich des Hochvakuums und verlangt zur Langzeitstabilisierung oft die Verwendung von Getter- bzw. Trockner-Materialien für Gasmoleküle bzw. Wasserdampf.

Das Druckniveau des Vakuums darf stark erhöht werden, wenn offenporiges Material zwischen die Wandungen gefüllt wird und die vergleichsweise geringere Isolierwirkung akzeptabel ist. Die Effektivität der Isolierung reduziert sich weiter, wenn der aufgefüllte Bereich in einer weiteren Ausführungsform nicht evakuiert wird.

Einsätze von Kühlmitteln (z.B. mit sogenannten Phasenänderungsmaterialien) nahe der Innenwand des Behälters (z.B. eingelegt im Behälterinnenraum oder in einer zusätzlichen, hülsenförmigen Wand) sind möglich, vergrößern aber weiter den Durchmesser oder die Länge des Behälters.

Das Gehäuse und der bzw. die Deckel weisen vorzugsweise eine harte Oberfläche auf, um z.B. Kratzer durch andere Gegenstände in der Handtasche oder auch bei der Reinigung mit Bürsten zu vermeiden. So bietet sich z.B. die anodische Oxidation der Oberfläche (Eloxieren) bei Verwendung von Aluminium oder dessen Legierungen an.

Andere Metalle mit höherer Zugfestigkeit im Vergleich zu Aluminium oder Aluminiumlegierungen können die Oberflächenhärte weiter erhöhen. Hier kommen z.B. insbesondere Titan und dessen Legierungen und Edelstähle als Behältermaterial in Betracht. Je nach Werkstoff lassen sich nebst Eloxieren auch weitere Härtesteigerungen erreichen, wie z.B. durch Wärmebehandlung, Einbringen von Fremdatomen, Auftragen von Schutzschichten oder durch mechanische Verfestigung. Die anodische Oxidation von Titan oder Titanlegierungen erlaubt gleichzeitig die farbliche Gestaltung des Behälters aus diesem Material.

Für anspruchsvolle Umgebungen soll der Aufbewahrungsbehälter letztendlich möglichst wasserdicht und korrosionsbeständig sein, damit es z.B. im Regen, beim Schwimmen im Hallenbad, im Meer oder zur Reinigung mit kochendem Wasser oder in der Spülmaschine verwendet werden kann.

Der Aufbewahrungsbehälter ist bei aufgesetztem(n) Deckel(n) vorzugsweise hermetisch gegenüber der Umgebung verschlossen. Für die Dichtheit kann z.B. auf Höhe des Deckelanschlags auf dem Gehäuse ein Dichtungsring aus Kunststoff angebracht werden. Alternativ kann ein Dichtungsring auf der Behälterinnenseite des Deckelbodens eingesetzt werden, auf den dann der obere Rand der Gehäuseöffnung drückt.

Das Gehäuse und/oder der Deckel können aus Metall oder Kunststoff bestehen, insbesondere aus Titan oder einer Titanlegierung. Bei der Korrosionsbeständigkeit zeigen sich Titan sowie Titan- oder Edelstahllegierungen als besonders gut geeignet. Kunststoffe, wie z.B. Polyethylen, Polyproplyen oder Polyamide, sind eine kostengünstigere Alternative. Bestimmte Aluminiumlegierungen können für viele Anwendungen eine ausreichende Korrosionsbeständigkeit bieten.

Mehrere Aufbewahrungsbehälter können zu einem Bündel verkettet werden, z.B. indem diese in mehrere elastische Schlaufen eingeschoben werden.

Die vorliegende Erfindung wird nun anhand der folgenden Abbildungen näher beschrieben, ohne auf diese beschränkt zu sein:

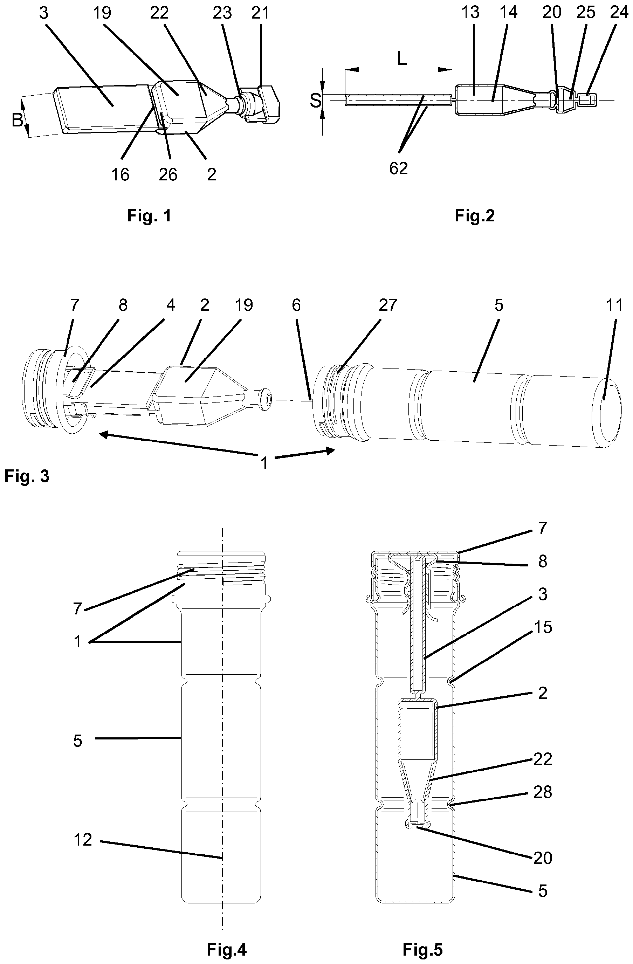

Fig. 1 zeigt eine Pipette 2 in räumlicher Ansicht und Fig.2 dieselbe Pipette in einer Schnittansicht in Längsrichtung 14. Die Pipette weist hintereinander angeordnet auf: eine flächige, greifbare Fahne 3, eine Abgabekammer 19, in der sich ein fließfähiges Behandlungsmedium 13 befindet, eine Abgabeöffnung 20 und eine Verschlusskappe 21 auf der Abgabeöffnung. Die Pipette ist etwa in der Mitte bauchig ausgestaltet, dort wo die Abgabekammer 19 ist, und verengt sich in Richtung auf die Abgabeöffnung 20. Der verengte Teil der Abgabekammer wird Halsstück 22 genannt. Das Ende des Halsstückes wird als Pipettenspitze 23 bezeichnet. Die Verschlusskappe 21 hat ein Griffstück 24 zum Trennen der Verschlusskappe von der Pipette und eine hohle Halbkugel 25 zum Wiederverschließen der Pipette.

Die Abgabekammer 19 geht an ihrem hinteren Ende 26 in das vordere Ende der Fahne 16 über. Die Fahne bildet relativ zur Abgabeöffnung 20 das gegenüberliegende Ende der Pipette. Die Fahne hat eine Plättchenform.

Die dargestellte Fahne hat die Form eines dünnen länglichen Quaders mit einer Breite B, einer Länge L und einer Stärke S. Die Fahne ist hohl und hat somit zwei großflächige Wandungen 62, die in Richtung der Stärke S federnd eingedrückt werden können. Die Fahne ist weiterhin elastisch biegbar um eine Achse parallel zur Richtung der Breite B.

Fig. 3 zeigt einen Aufbewahrungsbehälter 1 umfassend ein rohrförmiges Gehäuse 5 mit rundem Querschnitt, dessen erstes Ende ein außen umlaufenden Schraubgewinde 27 und eine erste Öffnung 6 aufweist und dessen zweites Ende durch einen Gehäuseboden 11 verschlossen ist. Ein abnehmbarer Deckel 7 ist auf das Schraubgewinde unter Drehen aufsetzbar, wobei der Deckel innenseitig eine Aufnahme 8 beinhaltet, in die das Fahnenende 4 einer Pipette 2 eingeschoben ist, wobei es sich in der Aufnahme festklemmt. Der Deckel 7 hat eine Topfform mit einem Innengewinde und ist vom Gehäuse 5 abgeschraubt. Die Pipette 2 ist geöffnet und ist aus dem Gehäuse 5 herausgezogen. Die Pipette wird in seitlicher Ansicht gezeigt (schmale Längsseite der Fahne), wobei die bauchige Abgabekammer 19 gut sichtbar wird.

Fig. 4 und ihre Schnittansicht Fig.5 zeigen den Aufbewahrungsbehälter 1 aus Fig.3 mit aufgesetztem Deckel 7, wobei der Deckel auf das Außengewinde des rohrförmigen Gehäuses 5 aufgeschraubt wurde. Die Pipette 2 ist mit der Aufnahme 8 sicher in dem Aufbewahrungsbehälter 1 gehaltert, wobei die Pipette zusätzlich durch eine erste umlaufende, radiale Verengung 15 des Gehäuses im Bereich der Fahne 3 parallel zur Längsachse 12 des Gehäuses ausgerichtet ist. Die Verengung dient demnach als seitliche Führung. Die zweite umlaufende Verengung 28 hat dann nur noch die Funktion, die Gehäusewand mechanisch zu stabilisieren und eine eventuelle Halterung des gesamten Behälters durch eine zusätzliche Konstruktion zu unterstützen. Das untere Ende des Halsstücks 22 und die Abgabeöffnung 20 sind allseitig von der Innenwand des rohrförmigen Behälters 1 beabstandet. Die Abgabeöffnung 20 ist nicht verschlossen. Trotzdem läuft keine Flüssigkeit aus.

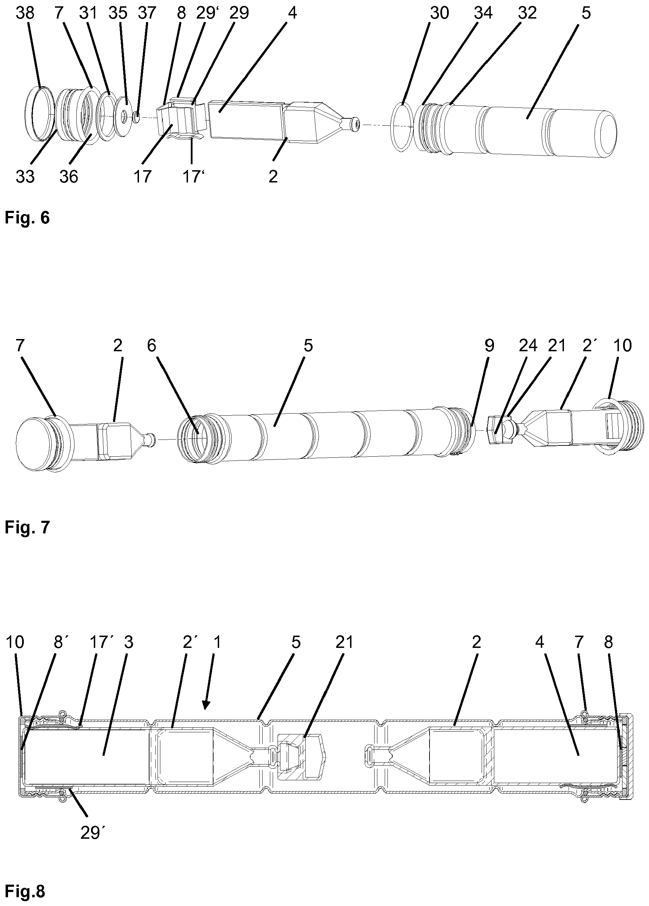

Fig. 6 zeigt in einer Explosionszeichnung einen Aufbewahrungsbehälter 1 mit rohrförmigem Gehäuse 5, Deckel 7, Aufnahme 8 und Pipette 2. Die Aufnahme 8 weist je eine Paarung aus Federwange 17 und 17' und zugehörigem Führungsanschlag 29 und 29' auf.

Als Erweiterung der obigen Ausführungen aus Fig.3 bis Fig. 5 werden nach der Ausführung gemäß Fig.6 zwei Dichtungsringe gezeigt: ein Dichtungsring 30 kann - bis zu dem Anschlagwulst 32 des Gehäuses 5 für den Deckelöffnungsrand- auf das Gehäuse geschoben werden. Ein weiterer alternativer Dichtungsring ist als Flachdichtungsring 31 im Deckelboden 33 eingelegt, wogegen die Kante der Gehäuseöffnung 34 beim Einschrauben des Deckels 7 gedrückt wird.

Als weitere Erweiterung der obigen Ausführungen aus Fig.3 bis Fig. 5 wird nach der Ausführung gemäß Fig.6 eine ringförmige Metallscheibe 35 an der Deckelinnenseite 36 mit dem Deckelboden 33 verbunden, worauf wiederum die Aufnahme 8 befestigt ist. Die Metallscheibe 35 dient als Gewicht bei sehr leichten Deckelkonstruktionen, damit der Deckel 7 mit eingeschobenem Fahnenende 4 und damit gehalterter Pipette 2 auf eine Ablagefläche mit seinem Deckelboden 33 stabil aufgestellt werden kann. Desweiteren nimmt die Metallscheibe einen kleinen Dauermagneten 37 zentrisch auf und verstärkt dessen Magnetfeld in Richtung der äußeren Deckelbodenseite. So ist das Anhaften des Deckels oder des ganzen Behälters an ferromagnetischen Komponenten wie z.B. im Bad oder Büro möglich. Um beim Tragen des Behälters 1 in sensibler Umgebung das Magnetfeld nach außen abzuschirmen, ist in dieser Ausführung eine zusätzliche ferromagnetische Kappe 38 auf der Außenseite des Deckelbodens 33 aufgesteckt.

Fig. 7 und 8 zeigen einen Aufbewahrungsbehälter 1 mit einem Gehäuse 5 in Form eines an beiden Enden offenen Rohres mit zwei aufschraubbaren Deckeln 7,10 für die erste 6 und zweite 9 Öffnung. In die Aufnahmen 8,8' beider Deckel ist jeweils eine Pipette 2,2' mit ihrem Fahnenende 4 eingesetzt, eine Pipette 2 davon ist im geöffneten Zustand, bei der anderen Pipette 2' ist die Verschlusskappe 21 noch nicht abgedreht worden. Dort ist auch das Griffstück 24 als Teil der Verschlusskappe 21 sichtbar.

Fig.8 zeigt einen Schnitt durch den Behälter 1 nach Fig. 7. Die Fahne 3 ist jeweils in einer Aufnahme 8,8' im Deckel 7,10, die zwei Federwangen 17 und 17' wie in Fig.6 aufweist, eingeklemmt. Eine Federwange drückt die Fahne 3 gegen eine ebene Fläche der Aufnahme, an die sich die Fahne anlegt und die die Fahne und damit die Pipette in Richtung der Drehachse des Schraubdeckels ausrichtet. Die ebene Fläche der Aufnahme fungiert als Führungsanschlag 29.

Zur Verbesserung des Tragekomforts kann die Gehäuselänge reduziert werden, indem die Fahnen in Ihrer Länge L vor der Anwendung des Behälters am hinteren Ende gekürzt werden (z.B. mit einer Schere), insbesondere auf eine verbleibende Fahnenlänge von 0,5 bis 1 cm. Eine weitere Reduktion der Gehäuselänge wäre möglich, wenn der Anwender eine oder beide Pipetten ohne Verschlusskappe verwenden mag.

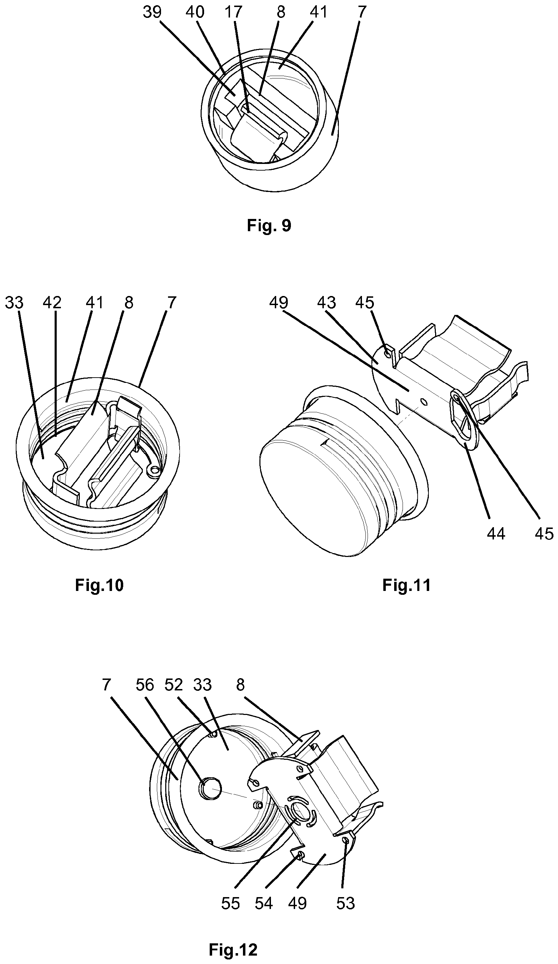

Fig. 9 zeigt einen Deckel 7 in einer räumlichen Darstellung, der für einen Schnappverschluss mit seinem zugehörigen Ende des Gehäuses ausgelegt ist. Man erkennt eine schlitzförmige Aufnahme 8, die das Fahnenende 4 aufnehmen und mit der einen Federwange 17 festhalten kann. Schiebt man die Fahne 3 ein, so gleitet das Fahnenende 4 an der engsten Stelle der Aufnahme vorbei, die Federwange 17 biegt sich leicht unter Federspannung nach hinten und liegt dann satt auf der Fahne auf. Die Führung in Richtung der Fahnenbreite erfolgt federlos durch ebene Führungsflächen 39. Dies kann in der Regel bei den engeren herstellerbedingten Abweichungen der Fahnenbreite mit leichtem Genauigkeitsverlust der Pipettenausrichtung akzeptabel sein. Der Deckel besitzt an der inneren Deckelseitenwand 41 eine ringförmige Verengung 40 für die Schnappverbindung mit dem Gehäuse. Der Deckel kann im einfachsten Fall im Spritzguss- oder Fließpressverfahren in einem Teil hergestellt werden, so dass die Aufnahme nicht separat gefertigt werden muss.

Fig. 10 und 11 zeigen eine Abwandlung der Aufnahme 8 nach den Fig. 3 bis 8. Für die Befestigung der Aufnahme am Deckelboden 33 wird die innere Deckelseitenwand 41 mit einer kreisförmig umlaufenden Nut 42 versehen. In diese Nut greifen die zwei seitlichen Flügel 43,44 des Bodens der Aufnahme ein. Ein Flügel 43 ist wie ein Kreissegment geformt, der zweite Flügel ist nach dem Prinzip eines Armes von einem Sicherungsring (auch Seeger-Ring genannt) 44 geformt. Alternativ - aber vorliegend nicht gezeigt - kann das Kreissegment durch einen zweiten Arm in punktsymmetrischer Anordnung (des ersten Arms zum Deckelboden-Mittelpunkt) ersetzt werden. Das Einsetzen der Aufnahme 8 in den Deckel 7 erfolgt mit der üblichen Fingerzange, deren Finger in die zwei Hilfsbohrungen 45 eingreifen und zusammengezogen werden. Dadurch wird der Sicherungsring-Arm 44 unter Vorspannung gesetzt. Dann wird die Aufnahme 8 in den Deckel 7 bis zum Anschlag auf den Deckelboden 33 eingeführt.

Dort schiebt man den Flügel 43 mit dem Kreissegment in die Deckelnut 42 und lässt auch den Arm der Sicherungsfeder 44 in diese Nut 42 schnappen. Um die Verwendung einer Spezialzange zu vermeiden kann auch die Form eines Lamellen-Sicherungsrings - hier nicht gezeigt - verwendet werden. Statt der Hilfsbohrungen 45 besitzt dieser Ring ein (zur Kreismitte hin) zugespitztes Ende, das ein späteres Lösen der Aufnahme 8 mit einem Schlitzschraubendreher ermöglicht.

In Fig. 12 ist die Aufnahme 8 bis auf den Aufnahmeboden 49 nahezu identisch zur Federkonstruktion in Fig.11. Der Aufnahmeboden bzw. Federboden 49 weist hier jedoch andere Durchbrüche auf. Wird der Deckel 7 nicht tiefgezogen, sondern durch Fließpressen hergestellt, dann lassen sich erhabene Formen auf der Deckelinnenseite des Deckelbodens beim Metallumformen realisieren. Vom Deckelboden 33 herausstehende Stifte fixieren dann die Aufnahme, zum einen durch eine Presspassung von den seitlichen Stiften 52 mit Löchern 53 im Federboden oder mit seitlichen Federboden-Löchern mit Schlitzen 54 oder zum anderen mit Hilfe eines einzigen, größeren zentrischen Lochs mit flexiblen Lochwänden 55 zur federnden Aufnahme des entsprechend großen Stiftes 56 auf dem Deckelboden. Letztendlich kann die Aufnahme 8 auch durch axial-radiale Verformung der Stiftköpfe (z.B. Drücken oder Hämmern) über die obere Oberfläche des Federboden-Bleches, ähnlich einer Nietverbindung, d.h. nicht wiederlösbar, gesichert werden. Stifte und Löcher hätten hier eine Spielpassung.

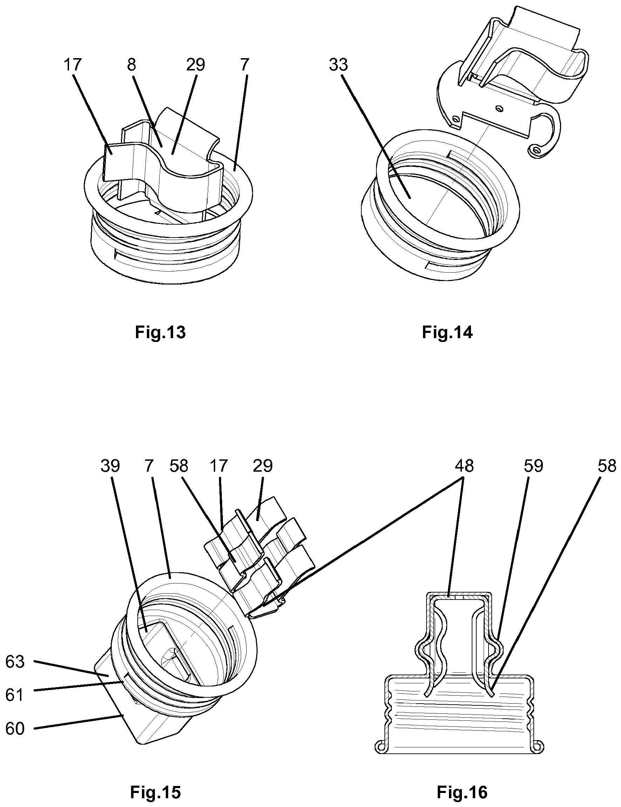

In Fig.13 und Fig.14 wird ein Deckel 7 mit einer Federkonstruktion als Aufnahme 8 versehen, wobei Deckel 7 und der Aufnahmeboden 49 der Darstellung in Fig.11 entsprechen. Abweichend davon wird hier auf eine Klemmung der schmalen Fahnenseite verzichtet. Außerdem wird für die Fahnenfixierung in der Aufnahme 8 eine Federwange 17 mit einer zum Deckelboden 33 parallelen statt vertikalen Biegeebene verwendet. Wenn die geschwungene Federwange 17 an ihrem Ende vom Führungsanschlag 29, z.B. mit dem Daumen, etwas weggezogen wird, kann das Fahnenende 4 der Pipette nahezu widerstandslos in die Aufnahme 8 eingeführt werden. Danach wird die Federwange 17 wieder losgelassen, schnappt auf die große Fahnenfläche 62 der Pipette 2 und fixiert so die Pipette im Deckel 7. Optional könnte eine zusätzliche nadelförmige Spitze an der Federwange, die im Bereich der Kontaktlinie zwischen Federwange und Fahne liegt, die Pipette stärker fixieren, indem sich die Spitze in die Fahne bohrt.

In Fig. 15 und 16 ist ein topfförmiger Deckel mit quaderförmiger Extrusion 63 gezeigt, wodurch die Fahne 3 der Pipette 2 soweit im Deckel 7 zum Verschwinden gebracht wird, dass das Drücken der Abgabekammer 19 gerade nicht gestört wird. Die größere Quaderbreite an der Aufnahmeöffnung ist der Breite der Pipettenfahne 3 angepasst, die zugehörigen Führungsflächen 39 führen die Pipette dementsprechend in dieser Richtung. Der Quader nimmt eine Feder 48 als Pipettenhalter auf, deren zwei mittige Federelemente 58 in die zwei kleinen seitlichen Auswölbungen der Quaderwände 59 in Form einer Schnappverbindung einrasten. Durch Zusammendrücken der Enden dieser Federelemente 58 ist die gesamte Feder wieder einfach aus dem Deckel 7 entfernbar, z.B. für eine Reinigung. Die beiden seitlichen Paarungen der Federkonstruktion aus Federwange 17 und Führungsanschlag 29 sind in ihrer Form analog zu den obigen Ausführungen gestaltet und richten die Pipette 2 in Richtung der Deckeldrehachse aus. Der gerade Führungsanschlag 29 kann durch geschwungene Federwangen 17 ersetzt werden. Ein Vorteil der Quaderkonstruktion gegenüber den Konstruktionen nach Fig. 10 bis 14 liegt in der hohen Steifigkeit der Aufnahme bei Torsion der Pipette, z.B. beim Abdrehen der Verschlusskappe 21, da das Torsionsmoment hauptsächlich von der Deckelwand und nicht von der Federkonstruktion 48 aufgenommen wird. Weitere Vorteile ergeben sich durch eine einfache Abdichtung mit einem Flachdichtungsring 31 im Deckelboden, das einfachere Aufdrehen bei einem Schraubverschluss, das einfachere Abziehen des Deckels im Fall eines Schnappverschlusses und durch das mögliche Hinlegen des Deckels mit montierter Pipette auf den Tisch, in dem der Deckel auf einer der breiten Kanten der obersten Quaderfläche 60 und einem Punkt 61 auf der Kante des äußeren kreisrunden Deckelbodens aufliegt.

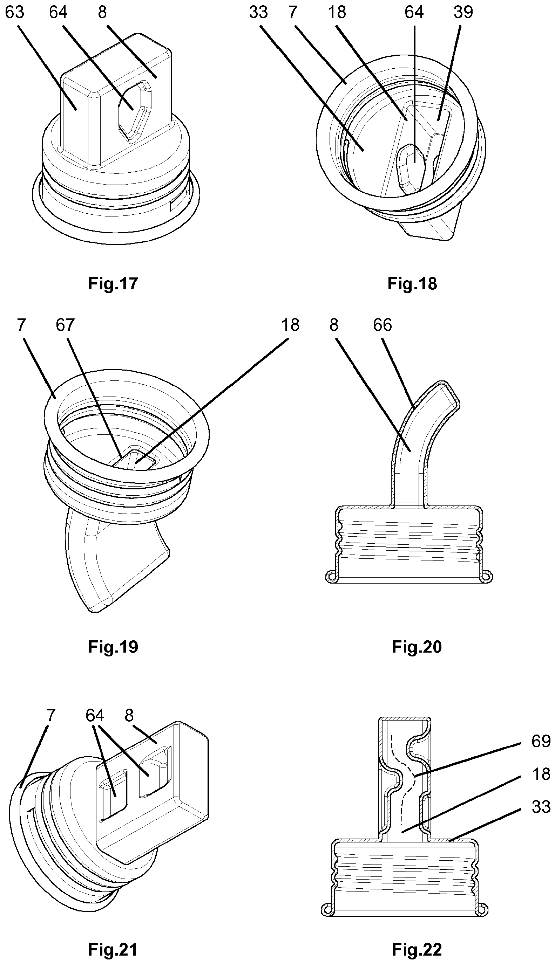

Die Fig.17 und Fig.18 zeigen die Form einer Aufnahme 8 im Deckel 7 in Form eines Mauls 18, in der die Federwirkung der großen Fahnenflächen 62 verwendet wird und es somit keiner weiteren Federelemente bedarf, wie in obigen Konstruktionen des Deckels. Wie im vorherigen Prinzip aus Fig.15 und Fig.16 hat der Deckelboden 33 eine quaderförmige Extrusion 63, die nah an den maximalen Abmessungen des Fahnenquerschnitts angepasst ist. An einer oder beiden großen Seitenwänden der Extrusion sind Vertiefungen 64 wie z.B. Sicken eingearbeitet, die ins Deckelinnere zeigen.

Der Abstand der Vertiefungen 64 ist kleiner als die Fahnenstärke S, so dass sich die Vertiefungen 64 in die großen Seitenflächen 62 der Pipettenfahne 3 hineindrücken, wenn das Fahnenende 4 in das Maul 18 geschoben wird. Eine Keilform der Vertiefungen verringert den Widerstand der hinteren Fahnenkante beim Einschieben der Fahne und verringert außerdem auch die Gefahr, dass Teilchen von einer möglichen Etikette an der Fahne abgerissen werden. Letzteres kann bei der Verwendung einer einzigen statt zweier Vertiefungen bzw. Sicken vermieden werden.

In Fig.19 und Fig.20 zeigen einen Deckel 7, bei dem die Eigenschaft der Pipettenfahne 3 genutzt wird, dass die Fahne um eine Achse parallel zur Richtung der Fahnenbreite B flexibel ist, ähnlicher einer Blattfeder. So besteht die Aufnahme 8 hier in der einfachen Form als Maul 18 mit einer bogenförmig gekrümmten Extrusion 66 am Topfdeckelboden. Der Boden hat eine rechteckige Öffnung 67, die möglichst eng an den Abmessungen des Fahnenquerschnitts angepasst ist. Dort wird das Fahnenende 4 eingeschoben und zwingt die Fahne 3 zu einer Krümmung. Die Krümmung erzeugt eine Biegekraft, die die Fahne an die Extrusionswand drückt. Durch die Biegekraft wirkt eine Reibkraft auf die Fahnenwand, die die Pipette in der Aufnahme 8 hält. Generell ist diese Konstruktion sehr robust, wenn die Verschlusskappe 21 der Pipette im eingeschobenen Zustand der Pipette abgerissen werden soll.

Nach einer weiteren in Fig.21 und Fig.22 dargestellten Ausgestaltung der Deckelkonstruktion ist die Aufnahme 8 in Form eines Mauls 18 kastenförmig auf den Deckelboden 33 aufgesetzt. Der sich im Kasteninneren durch Vertiefungen 64 an den Kastenwänden bildende, wellenförmige Gang 69 ist dreimal entgegengesetzt gekrümmt, so dass sich das Fahnenende 4 im wellenförmigen Gang 69 beim Einschieben festklemmt. Hier werden zwei Klemmmechanismen miteinander kombiniert: die Federwirkung der großen Fahnenflächen 62 (wie in Fig.17 und Fig.18) und die Federwirkung der Fahne durch mehrfaches Biegen um Achsen in Richtung der Fahnenbreite B.

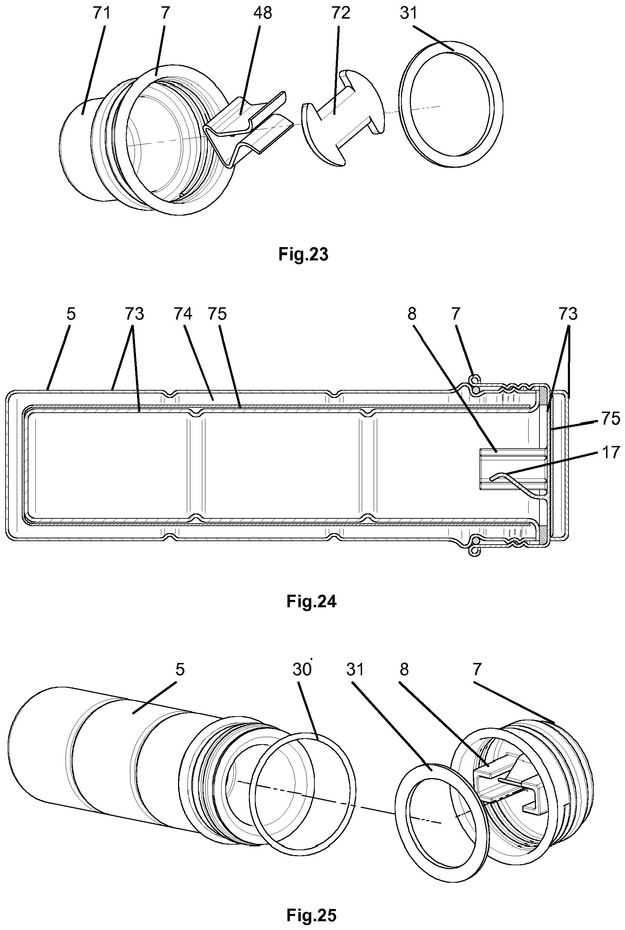

Die Fig.23 zeigt eine Variante der Aufnahme 8 mit einer einfachen Federkonstruktion, bei der die Feder 48 z.B. aus einem simplen Blechstreifen geformt werden kann. Eine zusätzliche zylindrische Extrusion 71 im Deckelboden 33 ist mit seinem Durchmesser der Fahnenbreite angepasst und zentriert die Pipette 2 so in entsprechender Querrichtung.

Die zusätzliche Extrusion 71 kann auf die dem Deckel 7 zugehörige Gehäuseöffnung 6 gesteckt werden. Die Feder 48 ist an den Deckel z.B. geklebt, gelötet oder geschweißt. Alternativ zu diesen Fügeverfahren wird hier ein zusätzliches Element 72 verwendet, um die Feder 48 in die zusätzliche zylindrische Extrusion 71 hinein zu pressen. Der gezeigte Flachdichtungsring 31 liegt auf dem Deckelboden vor der zusätzlichen Extrusion auf.

Die Fig.24 bis 26 zeigen eine Konstruktionsvariante des Aufbewahrungsbehälters 1, die eine Verwendung in wärmerer oder kalter Umgebung über längere Zeit durch thermische Isolierung der Außenwände des Behälters ermöglicht.

Zur Isolierung ist eine doppelte Wandung 73 des Behälters mit dazwischen liegendem Vakuum 74, wie in der Fig.24 als Schnittansicht gezeigt, konstruiert. Jeweils zwei Wände für das Gehäuse 5 und den Deckel 7 werden miteinander verschweißt.

Zur Reduzierung der Erwärmung durch Strahlung ist eine Superisolation (Vielschichtisolierung) 75 zwischen den Innen- und Außenwänden von Gehäuse und Deckel verwendet worden. Dazu eignen sich dünne gestapelte, metallbeschichtete Folien auch in Kombination mit eingelagerten Mikroteilchen zur Streuung oder Absorption von Infrarotstrahlung. Alternativ können reine Aluminiumfolien verwendet werden, die z.B. mit dünnem Gewebe als Abstandhaltern getrennt werden. Ergänzend können die Metalloberflächen des Behälters poliert oder spiegelnd bedampft werden.

Der für die Isolierung benötigte, erhöhte Durchmesser des Behälters wird in dieser Ausführung ausgenutzt, um den Deckel als Fließpressteil mit Innengewinde kompakt zu konstruieren, wie in Fig.25 gezeigt. Die Federwange 17 der Aufnahme 8 wird dabei in einem separaten Verfahrensschritt geformt.

Fig.26 zeigt nochmal die einzelnen Komponenten dieser Ausführung eines thermisch isolierten Behälters in einer Explosionszeichnung.

Die Fig.27 und 28 lehnen sich an die Konstruktion der Fig.24 bis 26 an. Hier wird die doppelwandige Konstruktion dadurch vereinfacht, dass -statt der Verwendung einer geschwungenen Federwange 17- eine Vertiefung 64 in die Wand der Aufnahme geformt ist, so wie bereits in Fig.17 beschrieben.

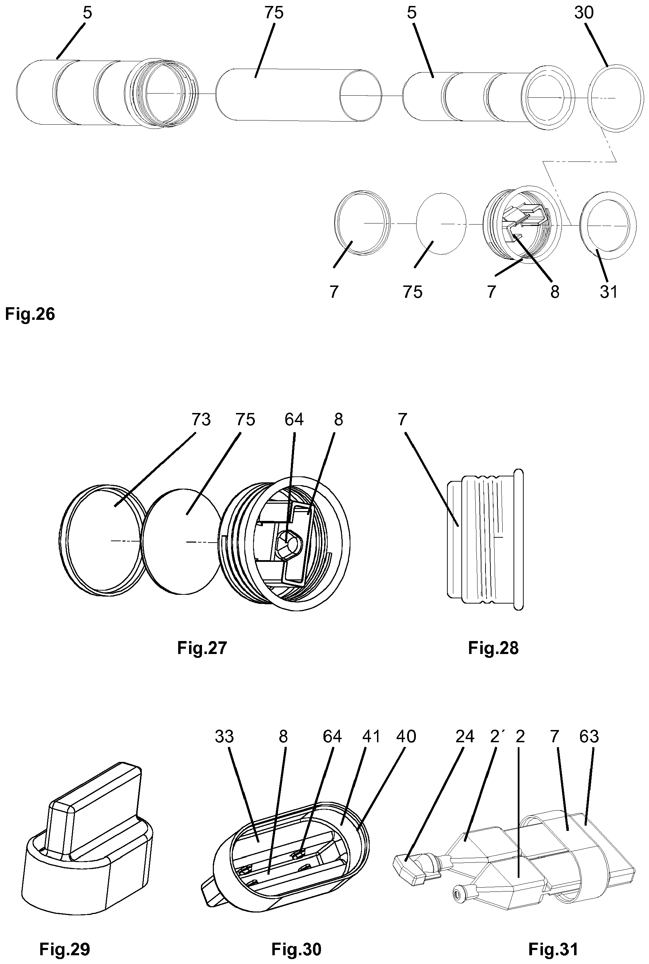

Die Fig. 29 bis 31 zeigen einen Deckel 7, der eine breite Aufnahme 8 für die Halterung von ein oder zwei Pipetten 2,2' besitzt. Die Klemmung erfolgt mit Hilfe von Vertiefungen 64 an einer quaderförmigen Extrusion 63 aus dem Deckelboden 33, so wie bereits in Fig.17 beschrieben. Der Deckel besitzt an der inneren Deckelseitenwand 41 eine Verengung 40 für die Schnappverbindung mit dem Gehäuse. Werden zwei Pipetten aufgenommen, liegen diese im Deckel auf einer ihrer schmalen Längsseiten direkt nebeneinander, wie in Fig.31 gezeigt. Dabei können sie getrennt voneinander oder noch mit Verbindungsstegen als Streifen verbunden sein. Fig.31 zeigt die erste Pipette 2 geöffnet und die zweite Pipette 2' ungeöffnet. Das Drücken der Abgabekammer der geöffneten Pipette wird durch die ungeöffnete Pipette nicht behindert. Das Tropfen der Behandlungsflüssigkeit geschieht ungestört, solange das Griffstück 24 nicht allzu lang ist.

### Bezugszeichenliste:

| | | | |
|---|---|---|---|
| Aufbewahrungsbehälter / Behälter | 1 | Ringförmige Metallscheibe | 35 |
| Pipette | 2 | Deckelinnenseite | 36 |
| Fahne / Pipettenfahne | 3 | Dauermagnet | 37 |
| Fahnenende / Hinteres Ende der Fahne | 4 | Ferromagnetische Kappe | 38 |
| Gehäuse / rohrförmiges Gehäuse | 5 | Führungsfläche | 39 |
| Erste Öffnung | 6 | Ringförmige Verengung | 40 |
| Deckel / Erster Deckel | 7 | Innere Deckelseitenwand | 41 |
| Aufnahme / Pipettenaufnahme | 8 | Kreisförmig umlaufende Nut / Deckelnut | 42 |
| Zweite Öffnung | 9 | Flügel mit dem Kreissegment | 43 |
| Zweiter Deckel | 10 | Arm der Sicherungsfeder / Sicherungsring-Arm | 44 |
| Gehäuseboden | 11 | Hilfsbohrungen | 45 |
| Längsachse des Gehäuses | 12 | Feder / Federkonstruktion | 48 |
| fließfähiges Behandlungsmedium | 13 | Federboden / Aufnahmeboden | 49 |
| Längsachse der Pipette | 14 | Seitliche Stifte | 52 |
| Erste radiale Verengung des Gehäuses (auch zur Führung der Pipette) | 15 | Löcher im Federboden | 53 |
| Vorderes Ende der Fahne | 16 | Seitliche Federbodenlöcher mit Schlitzen | 54 |
| Federarme / Federwange | 17 | Zentrisches Loch mit flexiblen Lochwänden | 55 |
| Maul | 18 | Großer Stift | 56 |
| Abgabekammer | 19 | Mittige Federelemente | 58 |
| Abgabeöffnung | 20 | Seitliche Auswölbungen der Quaderwände | 59 |
| Verschlusskappe | 21 | Breite Kante der obersten Quaderfläche | 60 |
| Halsstück | 22 | Punkt auf der Kante des äußeren kreisrunden Deckelbodens | 61 |
| Pipettenspitze | 23 | Große Fahnenfläche / großflächige Wandung | 62 |
| Griffstück | 24 | Quaderförmige Extrusion | 63 |
| Hohle Halbkugel | 25 | Vertiefungen / Sicken | 64 |
| Hinteres Ende der Abgabekammer | 26 | Gekrümmte Extrusion | 66 |
| Schraubgewinde | 27 | Rechteckige Öffnung | 67 |
| Zweite radiale Verengung des Gehäuses | 28 | Wellenförmiger Gang | 69 |
| Führungsanschlag | 29 | Zylindrische Extrusion | 71 |
| Dichtungsring | 30 | Zusätzliches Element | 72 |
| Flachdichtungsring | 31 | Doppelte Wandung | 73 |
| Anschlagwulst | 32 | Vakuum | 74 |
| Deckelboden | 33 | Superisolation/Vielschichtisolierung | 75 |
| Kante der Gehäuseöffnung | 34 | | |

## Patentansprüche

1. Aufbewahrungsbehälter (1) für zumindest eine Pipette (2) mit Fahne (3), wobei die Fahne ein Ende der Pipette bildet und die Fahne ein Fahnenende (4) aufweist, wobei der Aufbewahrungsbehälter aufweist:
- ein rohrförmiges Gehäuse (5), das an seinem ersten Ende eine erste Öffnung (6) aufweist und
- einen ersten Deckel (7) zum Verschließen und Öffnen der ersten Öffnung (6) des Gehäuses (5), wobei der Deckel auf seiner der Öffnung des Gehäuses zugewandten Seite eine Aufnahme (8) für das Fahnenende (4) der Pipette zum Haltern der zumindest einen Pipette am Deckel aufweist.

2. Aufbewahrungsbehälter (1) nach Anspruch 1, wobei das rohrförmige Gehäuse (5) an seinem zweiten Ende
a) eine zweite Öffnung (9) aufweist und der Aufbewahrungsbehälter (1) einen zweiten Deckel (10) zum Verschließen und Öffnen der zweiten Öffnung (9) aufweist, oder
b) einen Gehäuseboden (11)
aufweist,
wobei der zweite Deckel (10) vorzugsweise auf seiner der zweiten Öffnung (9) des Gehäuses (5) zugewandten Seite eine weitere Aufnahme (8') für das Fahnenende (4) einer Pipette (2,2') zum Haltern der Pipette (2,2') am zweiten Deckel aufweist.

3. Aufbewahrungsbehälter (1) nach zumindest einem der vorhergehenden Ansprüche, wobei das erste Ende und das zweite Ende des Gehäuses (5) sich entlang der Längsachse (12) des Gehäuses gegenüberliegen.

4. Aufbewahrungsbehälter (1) nach zumindest einem der vorhergehenden Ansprüche, wobei die zumindest eine Pipette (2) im/am ersten Deckel (7) gehaltert ist und wenn die Öffnung (6) mit dem ersten Deckel verschlossen ist, die zumindest eine Pipette (2) vom Gehäuse (5) umschlossen wird, wobei die Längsachse (14) der ersten Pipette (2) im Wesentlichen parallel zur Längsachse (12) des rohrförmigen Gehäuses (5) ausgerichtet ist, vorzugsweise koaxial und unabhängig hiervon vorzugsweise die Pipette (2), zumindest ab dem vorderen Ende der Aufnahme (8) im Wesentlichen freischwebend, von der Aufnahme (8) gehaltert ist, abgesehen von etwaigen seitlichen Führungen im Inneren des Gehäuses.

5. Aufbewahrungsbehälter (1) nach zumindest einem der vorhergehenden Ansprüche, wobei der erste Deckel (7) oder der erste Deckel (7) und der zweite Deckel (10) jeweils mit Pipette (2,2') in der Aufnahme (8,8') und ohne Pipette in der Aufnahme (8,8') von dem Gehäuse (5) abnehmbar und vorzugsweise ohne das Gehäuse frei beweglich ist/sind.

6. Aufbewahrungsbehälter (1) nach zumindest einem der vorhergehenden Ansprüche, wobei die Länge des Aufbewahrungsbehälters 3 bis 15 cm und die Breite 1 bis 4 cm beträgt.

7. Aufbewahrungsbehälter (1) nach zumindest einem der vorhergehenden Ansprüche, wobei die Aufnahme (8) durch eine oder mehrere Federwangen (17) zum klemmenden Haltern des Fahnenendes (4) ausgebildet ist und die Aufnahme (8) ggf. weiterhin zumindest einen Führungsanschlag (29) und/oder zumindest eine Führungsfläche (39) aufweist.

8. Aufbewahrungsbehälter (1) nach zumindest einem der vorhergehenden Ansprüche, wobei die Aufnahme in Form eines Mauls (18) ausgebildet ist, wobei das Fahnenende (4) klemmend in das Maul (18) einschiebbar ist und das Maul (18) vorzugsweise in Einschubrichtung einen wellen- oder S-förmigen Gang hat, einmal bogenförmig gekrümmt ist, zumindest einen Widerhalt in Form zumindest einer Vertiefung (64) in oder an der Maulwand hat und/oder sich das Maul nach hinten klemmend verengt.

9. Aufbewahrungsbehälter (1) nach zumindest einem der vorhergehenden Ansprüche, wobei das Gehäuse (5) und evtl. auch der Deckel (7) doppelwandig und/oder thermisch isolierend ausgebildet ist.

10. Set aufweisend zumindest den Aufbewahrungsbehälter (1) nach zumindest einem der vorhergehenden Ansprüche und zumindest eine Pipette (2) mit Fahne (3), wobei die Fahne (3) ein Ende der Pipette (2) bildet und das Fahnenende (4) der Fahne (3) in der Aufnahme (8) des Deckels (7,10) gehaltert ist.

11. Set aufweisend zumindest den Aufbewahrungsbehälter (1) nach zumindest einem der vorhergehenden Ansprüche, wobei die Fahne (3) die Form eines länglichen, flachen Quaders hat.

12. Set nach Anspruch 10, wobei der Aufbewahrungsbehälter (1)
a) eine Pipette (2) parallel zur Längsachse (12) des Gehäuses (5) enthält und das Gehäuse (5) mit einem Deckel (7) und einem Gehäuseboden (11) oder zwei Deckeln (7,10) verschlossen ist, wobei zumindest ein Deckel (7) eine Aufnahme (8) für das Fahnenende (4) der Pipette (2) aufweist; oder
b) zwei Pipetten (2,2') hintereinander und entlang der Längsachse (12) des Gehäuses (5) enthält und zwei Deckel (7,10) das Gehäuse (5) verschließen mit jeweils einer Aufnahme (8,8') für das Fahnenende (4) der jeweiligen Pipette (2,2'), wobei die Aufnahmen (8,8') verschieden sein können; oder
c) zwei Pipetten (2,2') parallel nebeneinander und parallel zur Längsachse (12) des Gehäuses (5) enthält und das Gehäuse (5) mit einem Deckel (7) und einem Gehäuseboden (11) oder mit zwei Deckeln (7,10) verschlossen ist, wobei der erste Deckel (7) eine Aufnahme für die Fahnenenden (4) von bis zu zwei Pipetten oder zwei Aufnahmen für das Fahnenende (4) von jeweils einer Pipette aufweist und wobei der zweite Deckel (10) keine Aufnahme besitzt.

13. Set nach zumindest einem der vorhergehenden Ansprüche 10 bis 12, wobei die Pipette (2, 2') eine Abgabekammer (19) aufweist, in der sich eine Behandlungsflüssigkeit befindet, insbesondere für Augen, wobei die Abgabekammer (19) vorzugsweise 0,1 bis 1,5 ml, insbesondere 0,2 bis 0,6 ml, Behandlungsflüssigkeit enthält.

14. Set nach zumindest einem der vorhergehenden Ansprüche 10 bis 13, wobei das der Fahne (3) gegenüberliegende vordere Ende der Pipette (2) von einer Verschlusskappe (21) gebildet wird, die die Abgabekammer (19) verschließt und die Verschlusskappe (21) vorzugsweise im Zusammenhang mit dem ersten Gebrauch entfernt wurde oder nach dem Entfernen ggf. wieder aufgesetzt wurde.

15. Set nach zumindest einem der vorhergehenden Ansprüche 10 bis 14, wobei die Pipette (2, 2') aus einem Kunststoffmaterial gefertigt ist, insbesondere zwei zusammengefügten flächigen Kunststoffmaterialen, in denen zumindest die Abgabekammer (19) in Form von Halbschalen ausgeformt ist und vorzugsweise die Fahne von zwei planparallelen, flächigen Abschnitten der Kunststoffmaterialen gebildet wird.
